# EUROPÄISCHE PATENTSCHRIFT

(11) **EP 0 360 791 B1**
(45) Veröffentlichungstag und Bekanntmachung des Hinweises auf die Patenterteilung: **31.08.1994**
(21) Anmeldenummer: 89890246.5
(22) Anmeldetag: 21.09.1989
(51) Int. Cl.: A61B 17/22

(54) **Thrombektomie-Katheter**
Thrombectomy catheter
Cathéter de thrombectomie

(30) Priorität: 21.09.1988 AT 2316/88
(43) Veröffentlichungstag der Anmeldung: 28.03.1990
(73) Patentinhaber: Kaliman, Josef, Dr., A-1180 Wien (AT)
(72) Erfinder: Wichart, Alfred, A-1070 Wien (AT); Kaliman, Josef, Dr., A-1180 Wien (AT)
(74) Vertreter: Müllner, Erwin, Dr.

(56) Entgegenhaltungen:
- EP-A- 0 086 048
- EP-A- 0 254 414
- WO-A-80/02499
- US-A- 4 664 112

## Beschreibung

Die Erfindung betrifft einen Thrombektomie-Katheter zur Vergrößerung des Durchtrittsquerschnittes bei durch Arteriosklerose verengten Arterien mit Hilfe einer als Schlauch ausgebildeten Sonde die längs einer in die Arterie einfädelbaren Führungssonde nachschiebbar ist und eines stirnseitig am Schlauch vorgesehenen, von einer biegsamen Antriebswelle elektromotorisch angetriebenen Werkzeuges, insbesondere eines Fräsers oder Bohrers, wobei die Führungssonde, die gegebenenfalls aus mehreren Einzeldrähten bzw. Drahtkombinationen gebildet ist, mindestens eine axiale Ausnehmung im Werkzeug durchsetzt.

Zur exakten Führung und Positionierung eines Thrombektomie-katheters wird in die betroffene Arterie vorerst eine Führungssonde eingeschoben. Der Vorschub wird dabei ständig am Röntgenschirm kontrolliert. Bei einer bekannten Ausführung eines Trombektomie-Katheters wird, sobald die Führungssonde die Arterienverengung oder einen Verschluß erreicht hat, ein Schlauch nachgeschoben, an dessen Spitze ein mit einer axialen Bohrung ausgestattetes Werkzeug vorgesehen ist. Es wird sodann die Führungssonde aus der Arterie herausgezogen und eine biegsame Antriebswelle in den Schlauch eingeschoben. Die Antriebswelle trägt stirnseitig ein Kupplungsstück, das in ein entsprechendes Gegenstück am Werkzeug einrastet. Sobald die Antriebswelle mit dem Werkzeug gekuppelt ist, wird das Werkzeug in Rotation versetzt und zusammen mit einem über den Schlauch aufgebrachten Vorschub beginnt der eigentliche Räumvorgang. Dieser Thrombektomie-Katheter ist zwar äußerst wirkungsvoll, doch sind mit dem Ausfädeln der Führungssonde und mit dem Einfädeln der biegsamen Antriebswelle Verzögerungen und Gefahren verbunden, welche zu vermeiden, Aufgabe der Erfindung ist.

Ein weiterer aus der US-PS 4 700 705 bekannter Thrombektomie-Katheter umfaßt ein Werkzeug am stirnseitigen Ende eines Schlauches. Das Werkzeug trägt eine zylindrische Axialbohrung,durch welche eine Führungssonde schiebbar ist. Der Antrieb des Werkzeugkopfes erfolgt mit einer getrennten Antriebswelle koaxial zur Führungssonde. Der Aufbau dieser Vorrichtung ist äußerst kompliziert. Insbesondere ist infolge der komplexen Mechanik kein Freiraum für eine Zirkulation des Blutes bzw. für den Abtransport des abgetragenen Materials.

Ähnliche Probleme ergeben sich auch mit dem Katheter nach der EP-A3 191 630, bei der der Antrieb des Werkzeuges über eine unmittelbar am Werkzeug vorgesehene Turbine erfolgt.

Ein im Aufbau und in der Handhabung verbesserter und dadurch besonders sicher arbeitender Thrombektomie-Katheter ist gemäß der Erfindung dadurch gekennzeichnet, daß die Führungssonde gleichzeitig als Antriebswelle bzw. als Teil derselben ausgebildet ist und entweder in der Ausnehmung des Werkzeuges drehfest und axial verschiebbar geführt ist oder im Schlauch ein zur Führungssonde paralleler und am Werkzeug angreifender Draht vorgesehen ist, der bei Ubertragung des Drehmomentes mit der Führungssonde zu einer Antriebswelle verdrillt ist. Die Führungssonde kann dadurch im Inneren des Schlauches verbleiben. Durch die von der Kreisform abweichende Querschnittsform der Führungssonde bzw. die allenfalls mehrere Drähte mit Kreisquerschnitt oder Drahtkombinationen einhüllende Gesamtquerschnittsform und infolge der im Querschnitt übereinstimmenden, durchgehenden Ausnehmung im Werkzeug wird schon beim Aufschieben des Katheters auf die Führungssonde außerhalb des Patienten eine sichere und unmittelbar kontrollierbare drehfeste Kupplung zwischen der Führungssonde und dem Werkzeug erreicht, wobei die Verschiebbarkeit in axialer Richtung unbehindert erhalten bleibt. Das Innere des Werkzeuges, sowie insbesondere des Schlauches, steht infolge der einfachen Konstruktion für die Zirkulation bzw. Ableitung des Blutes bzw. des abgetragenen Materials weitgehend zur Verfügung. Dies gilt auch für die Ausführung, bei der parallel zur Führungssonde im Inneren des Schlauches ein zusätzlicher, am Werkzeug angreifender Draht vorgesehen ist. Die Führungssonde kann sodann, wenn der Katheter aufgeschoben ist, mit dem elektromotorischen, drehzahlregelbaren Antrieb gekuppelt werden und übernimmt dadurch gegebenenfalls zusammen mit dem zusätzlichen Draht die Funktion einer Antriebswelle für das Werkzeug. Der Vorschub des Thrombektomie-Katheters erfolgt während der Rotation des Werkzeuges gemeinsam mit der Führungssonde. Diese kann mit ihrem vorderen Ende über die Stirnfläche des Werkzeuges vortreten. Dadurch ergibt sich ein drehender Suchfinger, der den Weg durch die Verengung der Arterie vorgibt. Der hier als Suchfinger bezeichnete überstehende Teil der Führungssonde arbeitet dabei selbst als Werkzeug. Eine besondere Ausführungsform ist dadurch gekennzeichnet, daß die Führungssonde einen Rechteckquerschnitt aufweist und zur Förderung der flüssigen Phase sowie der abgetragenen Feststoffe in Richtung zum Ausgang des Schlauches zu einer Schraubenfläche tordiert ist. Eine Schraubenfläche stellt sich bei einer bandförmigen Führungssonde bei der Drehung ein. Besonders zweckmäßig ist es, eine bereits als Schraubenfläche ausgebildete Führungssonde zu verwenden und die Antriebsrichtung so zu wählen, daß sich eine nach außen gerichtete Förderwirkung ergibt. Es ist ferner vorteilhaft, wenn die Führungssonde aus mindestens zwei beieinanderliegenden, jeweils Kreisquerschnitt aufweisenden Einzeldrähten aufgebaut ist. Dabei können als Einzeldrähte zwei handelsübliche Sonden allenfalls unterschiedlichen Durchmessers verwendet werden. Es handelt sich dabei meist um Drähte, die selbst mit dünnem Draht eng umwickelt sind. Die beiden Einzeldrähte liegen aneinander und haben zusammen die Querschnittsform der Ziffer "8". Eine durchgehende Ausnehmung im Werkzeug hat ebenfalls diese Form. Es kann einer der Drähte auch etwas länger als der oder die anderen sein und die Funktion eines überstehenden Suchfingers übernehmen, während der oder die anderen Drähte nur bis zur Stirnfläche des Werkzeuges geführt sind. Die Drähte sind unabhängig voneinander axial verschiebbar. Sobald die Drähte in Rotation versetzt werden, legen sie sich schraubenlinienförmig aneinander. Dadurch können hohe Drehmomente übertragen werden. Der längere der beiden letztgenannten Drähte stellt die Führungssonde dar, der kürzere endet beim Werkzeug und greift an diesem an.

Bei einer anderen Ausführungsform sind die Einzeldrähte, die die Führungssonde aufbauen, miteinander verdrillt und bilden eine Schraubenfläche mit Förderrichtung zum Ausgang der Schlauches. Die Anordnung hat die Wirkung einer Rohrförderschnecke. Die Führungssonde ist antriebsseitig in einem Mitnehmer drehfest und längsverschiebbar gelagert und der Mitnehmer ist von einem Elekromotor antreibbar.

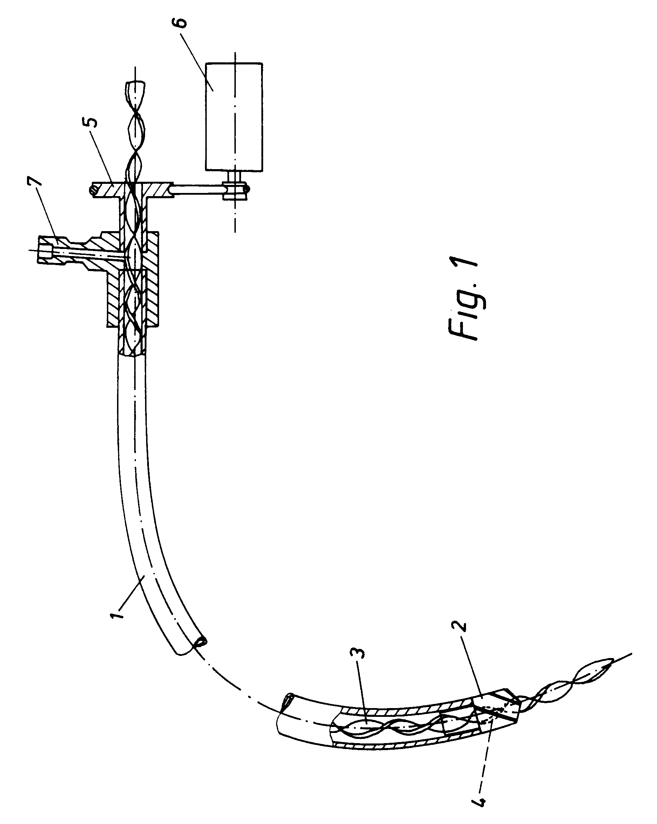

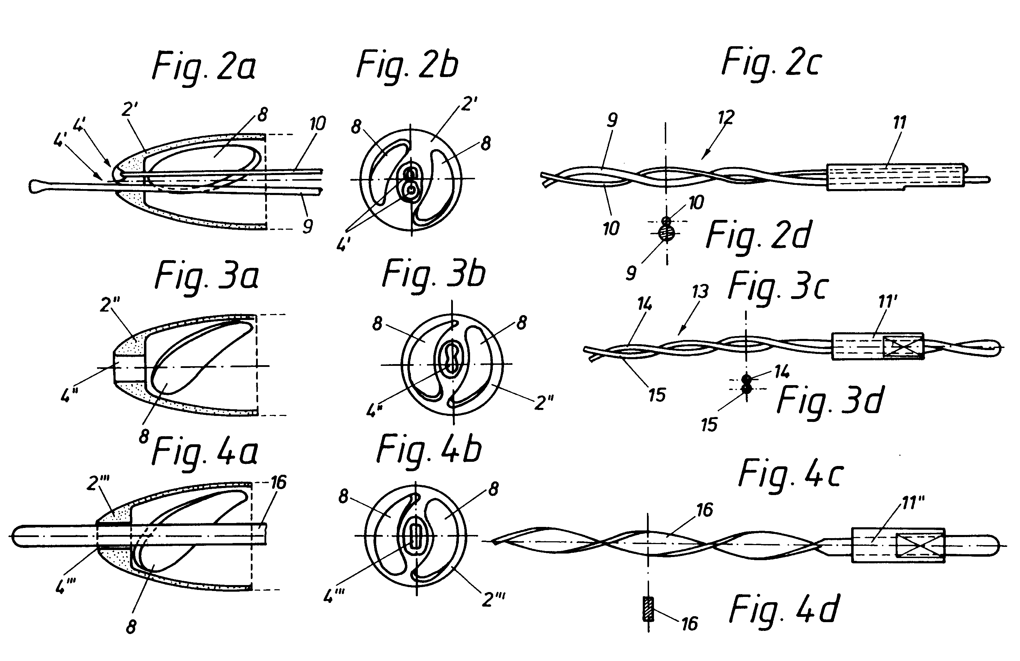

Ausführungsbeispiele des Erfindungsgegenstandes sind in den Zeichnungen dargestellt. Fig. 1 zeigt einen Thrombektomie-Katheter zusammen mit der Antriebseinheit, Fig. 2a einen Querschnitt durch einen Fräser, Fig. 2b eine Frontansicht des Fräsers, Fig. 2c eine Seitenansicht der Antriebswelle sowie des antriebsseitigen Mitnehmers, Fig. 2d einen Querschnitt durch die Antriebswelle und die Fig. 3a bis 3d sowie Fig. 4a bis 4d Alternativen zu Fig. 2a bis 2d.

Fig. 1 zeigt einen aus einem Schlauch 1 und einem am Schlauch stirnseitig drehbar angeordneten Werkzeug 2 (hier in Form eines Bohrers) bestehenden Thrombektomie-Katheters, wobei im Inneren des Schlauches 1 eine Führungssonde 3 vorgesehen ist. Die Führungssonde 3 wurde in eine Arterie eingeführt und der Schlauch 1 mit dem Werkzeug 2 nachgeschoben. Dazu weist das Werkzeug 2 eine axiale durchgehende Ausnehmung 4 auf. Die Führungssonde 3 hat eine von der Kreisform abweichende Querschnittsform, sie ist gemäß Fig. 1 im Querschnitt rechteckig und zu einer Schraubenfläche gewunden. Auch die Ausnehmung 4 bzw. ein Teil derselben hat diese Querschnittsform, sodaß sich eine drehfeste Kupplung zwischen Führungssonde 3 und Werkzeug 2 ergibt. An dem dem Werkzeug 2 gegenüberliegenden Ende des Thrombektomie-Katheters ist ein Mitnehmer 5 sowie ein elektromotorischer Antrieb 6 für die als Antriebswelle arbeitende Führungssonde vorgesehen. An einem Stutzen 7 liegt eine Pumpe (nicht dargestellt), die das Blut einschließlich des darin enthaltenen abgetragenen Materials der Arterienverengung absaugt. Nach entsprechender Aufarbeitung kann das Blut dem Kreislauf wieder zugeführt werden. Die sich drehende Schraubenfläche der Führungssonde 3 unterstützt die Förderung des Blutes in dem Schlauch 1. Die Drehrichtung des Antriebes 6 wird so abgestimmt, daß die Schraubenfläche in Richtung zum Stutzen 7 fördert. Das Werkzeug 2, 2′ 2˝ kann entsprechende Durchbrüche 8 (siehe Fig. 2a, 3a, 4a) aufweisen, welche das Absaugen durch den Schlauch hindurch ermöglichen.

Fig. 2c zeigt eine aus zwei Einzeldrähten 9, 10 bestehende Kombination 12. Die beiden Drähte, von welchen Draht 9 die eigentliche Führungssonde darstellt, können handelsübliche Sonden sein, die zugleich oder naheinander in die Arterie einschiebbar sind. Fig. 2c zeigt den Mitnehmer 11, an welchem der Antrieb 6 angreift. Fig. 2d zeigt den aus zwei Kreisquerschnitten zusammengesetzten Gesamtquerschnitt der Drahtkombination 12, die gemäß Fig. 2a und 2b in das Werkzeug drehfest, jedoch was den Draht 9 betrifft, längsverschiebbar eingreift (Ausnehmungen 4′). Die aus den beiden Drähten 9, 10 bestehende Kombination 12 kann so in die Arterie eingezogen werden, daß die beiden Drähte parallel laufen. Nach Aufschieben des Katheters und Einschalten des Antriebes 6 verdrillen sich die Drähte 9, 10 in der aus Fig. 2c ersichtlichen Weise. Der das Werkzeug tragende Teil der Sonde 9 arbeitet als Suchfinger und unterstützt ferner die abtragende Wirkung des Werkzeuges 2′.

Es kann aber, wie Fig. 3c zeigt, eine Führungssonde 13 verwendet werden, die aus bereits verdrillten Drähten 14, 15 besteht. Auch hier ist im Werkzeug 2˝ (Fig. 3a, 3b) eine formschlüssige Ausnehmung 4˝ vorgesehen.

Als weiteres Ausführungsbeispiel ist ein flexibles Flachband als Führungssonde 16 dargestellt. Dies kann zu einer Schraubenfläche verdreht sein bzw. unter dem Einfluß des über den Mitnehmer 11˝ wirkenden Antriebs 6 die Form einer Schraubenfläche annehmen. Im Werkzeug 2‴ ist eine dem Querschnitt angepaßte Ausnehmung 4‴ vorgesehen.

Wenn zum Aufbau der Führungssonde mehrere Einzeldrähte herangezogen werden, dann können diese jeweils für sich auch rechteckige wie auch kreisrunde Querschnitte aufweisen. Wesentlich ist, daß der einhüllende Gesamtquerschnitt als formschlüssige Kupplung zum Werkzeug wirkt und daher von der Kreisform abweicht, wobei der Führungssonde allein oder zusammen mit einem weiteren Draht 10 die Wirkung einer Antriebswelle zukommt.

## Patentansprüche

1. Thrombektomie-Katheter zur Vergrößerung des Durchtrittsquerschnittes bei durch Arteriosklerose verengten Arterien mit Hilfe einer als Schlauch ausgebildeten Sonde (1), die längs einer in die Arterie einfädelbaren Führungssonde (3, 9, 13, 16) nachschiebbar ist und eines stirnseitig am Schlauch vorgesehenen, von einer biegsamen Antriebswelle elektromotorisch angetriebenen Werkzeuges (2, 2', 2'', 2'''), insbesondere eines Fräsers oder Bohrers, wobei die Führungssonde, die gegebenenfalls aus mehreren Einzeldrähten bzw. Drahtkombinationen gebildet ist, mindestens eine axiale Ausnehmung (4, 4', 4'', 4''') im Werkzeug durchsetzt und über das Werkzeug (2, 2′, 2˝, 2‴) stirnseitig, beispielsweise 0,5 bis 2 cm zur Führung und Unterstützung der querschnittserweiternden Funktion des Werkzeuges distal vorschiebbar ist, dadurch gekennzeichnet, daß die Führungssonde (3, 9, 13, 16) gleichzeitig als Antriebswelle bzw. als Teil derselben ausgebildet ist und entweder in der Ausnehmung (4, 4′, 4˝, 4‴) des Werkzeuges (2, 2, 2˝, 2‴) drehfest und axial verschiebbar geführt ist oder im Schlauch (1) ein zur Führungssonde (9) paralleler und am Werkzeug angreifender Draht (10) vorgesehen ist, der bei Ubertragung eines Drehmomentes mit der Führungssonde (9) zu einer Antriebswelle verdrillt ist.

2. Thrombektomie-Katheter nach Anspruch 1, dadurch gekennzeichnet, daß die Führungssonde (16) einen Rechteckquerschnitt aufweist und zur Förderung der flüssigen Phase sowie der abgetragenen Feststoffe in Richtung zum Ausgang des Schlauches (1) zu einer Schraubenfläche tordiert ist.

3. Thrombektomie-Katheter nach Anspruch 1, dadurch gekennzeichnet, daß die Führungssonde (12, 13), wie bekannt, aus mindestens zwei beieinanderliegenden, jeweils Kreisquerschnitt aufweisenden Einzeldrähten (9, 10; 14, 15) aufgebaut ist.

4. Thrombektomie-Katheter nach Anspruch 3, dadurch gekennzeichnet, daß die Einzeldrähte (9, 10) der Führungssonde (12) gegeneinander axial verschiebbar sind.

5. Thrombektomie-Katheter nach Anspruch 3, dadurch gekennzeichnet, daß die Einzeldrähte (14, 15) im Sinne einer Schraubenfläche mit Förderrichtung zum Ausgang des Schlauches (1), wie bekannt, gegeneinander verdrillt ausgebildet sind.

6. Thrombektomie-Katheter nach einem der Ansprüche 1 bis 5, dadurch gekennzeichnet, daß die Führungssonde (3, 12, 13, 16) antriebsseitig, wie bekannt, in einem Mitnehmer (5, 11, 11′ 11˝) drehfest und längsverschiebbar gelagert ist und daß der Mitnehmer von einem Elektromotor (6) antreibbar ist.

## Claims

1. Thrombectomy catheter for enlarging the throughput diameter of arteries constricted as a result of arterial sclerosis with the aid of a probe (1) in the form of a tube which can be subsequently pushed along a guide probe (3, 9, 13, 16) which can be threaded into the artery and of a tool (2, 2', 2'', 2'''), provided on the end face of the tube, especially a cutter or drill, driven by an electric motor via a flexible drive shaft, wherein the guide probe, which is, if necessary, made of a plurality of separate wires or wire combinations, passes through at least one axial recess (4, 4', 4'', 4''') in the tool and for guiding and supporting the cross-sectional expansion operation of the tool can be pushed forward distally at the end face via the tool (2, 2', 2'', 2'''), for example 0,5 to 2 cm, **characterised** in that the guide probe (3, 9, 13, 16) is at the same time constructed as the drive shaft or as part of the same and is either guided non-rotatably and axially displaceably in the recess (4, 4', 4'', 4''') of the tool (2. 2', 2'', 2''') or in the tube (1) there is provided a wire (10), running parallel to the guide probe (9) and engaging the tool, which during torque transfer is twisted together with the guide probe (9) to form a drive shaft.

2. Thrombectomy catheter according to claim 1, **characterised** in that the guide probe (16) has a rectangular cross-section and is twisted to form a helical surface for conveying in the direction of the outlet of the tube (1) the liquid phase as well as the solid substances cleared away.

3. Thrombectomy catheter according to claim 1, **characterised** in that, as known, the guide probe (12, 13) is made up of at least two separate wires (9, 10; 14, 15), each having a circular cross-section, lying side by side.

4. Thrombectomy catheter according to claim 3, **characterised** in that the separate wires (9, 10) of the guide probe (12) are axially displaceable with respect to each other.

5. Thrombectomy catheter according to claim 3, **characterised** in that, as known, the separate wires (14, 15) are designed to be twisted with respect to each other in the sense of a helical surface conveying in the direction towards the outlet of the tube (1).

6. Thrombectomy catheter according to one of the claims 1 to 5, **characterised** in that, as known, the guide probe (3, 12, 13, 16) on the drive side is non-rotatably and longitudinally displaceably mounted in a follower (5, 11, 11', 11'') and that the follower can be driven by an electric motor (6).

## Revendications

1. Cathéter pour thrombectomie pour augmenter la section transversale de passage des artères rétrécies par l'artériosclérose, à l'aide d'un tube (1) réalisée sous la forme d'un tube flexible que l'on peut faire coulisser le long d'une sonde de guidage (3, 9, 13, 16) engagée comme un fil dans l'artère et d'un outil (2, 2', 2'', 2''') entraîné par un moteur électrique à partir d'un arbre d'entraînement flexible et prévu sur le côté frontal du tube flexible, en particulier d'une fraise ou d'un alésoir, la sonde de guidage, qui le cas échéant, est constituée de plusieurs fils individuels ou de combinaison de fils, traversant au moins un évidemment axial (4, 4', 4'', 4''') dans l'outil et étant susceptible de coulisser sur le côté frontal et en position distale sur l'outil (2, 2', 2'', 2'''), par exemple de 0,5 à 2 cm pour assurer le guidage et la bonne opération de la fonction d'élargissement de diamètre de l'outil, caractérisé en ce que la sonde de guidage (3, 9, 13, 16) est également réalisée pour constituer en même temps l'arbre d'entraînement ou une partie de ce dernier et est guidée dans la cavité (4, 4', 4'', 4''') de l'outil (2, 2', 2'', 2''') en étant immobile en rotation mais axialement déplaçable, ou en ce que dans la sonde flexible (1), un fil (10) disposé parallèlement à la sonde de guidage (9) et engagé avec l'outil à l'intérieur du tube flexible (1) est prévu, ce fil étant torsadé pour la transmission d'un couple de rotation par l'intermédiaire de la sonde de guidage (9) à un arbre d'entraînement.

2. Cathéter pour thrombectomie selon la revendication 1, caractérisé en ce que la sonde de guidage (16) comporte une section transversale rectangulaire et, tordue selon une surface hélicoïdale pour assurer la propulsion de la phase liquide ainsi que des matériaux solides extraits, dans la direction de la sortie du tube flexible (1).

3. Cathéter pour thrombectomie selon la revendication 1, caractérisé en ce que la sonde de guidage (12, 13), est réalisée, de façon connue avec, au moins deux fils individuels, (9, 10; 14, 15) disposés l'un à côté de l'autre et présentant chacun des sections transversales circulaires.

4. Cathéter pour thrombectomie selon la revendication 3, caractérisé en ce que les fils individuels (9, 10) de la sonde de guidage (12) sont susceptibles de coulisser axialement l'un par rapport à l'autre.

5. Cathéter pour thrombectomie selon la revendication 3, caractérisé en ce que les fils individuels (14, 15) sont réalisés de façon torsadée en sens contraire l'un de l'autre, de façon connue en soi, pour obtenir une surface hélicoïdale dont la direction de propulsion est dirigée vers la sortie du tube flexible (1).

6. Cathéter pour thrombectomie selon l'une des revendications 1 à 5, caractérisé en ce que la sonde de guidage (3, 12, 13, 16) est montée du côté d'entraînement, de façon connue en soi, solidaire en rotation et susceptible de coulisser longitudinalement dans un organe d'entraînement (5, 11, 11', 11'') et en ce que l'organe d'entraînement est susceptible d'être entraîné par un moteur électrique (6).
